# EUROPEAN PATENT APPLICATION

(11) **EP 4 368 160 A1**
(43) Date of publication of application: **15.05.2024**
(21) Application number: 22206479.2
(22) Date of filing: 09.11.2022
(51) Int. Cl.: A61F 13/15

(54) **FORMING POCKET SUITABLE FOR FORMING MOULDED ABSORBENT MATERIAL DEPOSIT STRUCTURE TO BE USED AS AN ABSORBENT CORE FOR ABSORBENT ARTICLES**

(71) Applicant: Ontex BV, 9255 Buggenhout (BE); Ontex Group NV, 9320 Erembodegem (BE)
(72) Inventor: HEEGE, Thomas, 56761 Düngenheim (DE); HOCHHAUSEN, Michael, 56727 Mayen (DE)
(74) Representative: Saurat, Thibault

(57) **Abstract**

The current invention concerns a forming pocket (10), suitable for forming moulded absorbent material deposit structures to be used as absorbent core for absorbent articles, the forming pocket (10), extending in a longitudinal, transversal and vertical direction (L,T,V), comprises:
- a grid structure (12) comprising a first set of grid members (18) and a second set of grid members (20), each set of grid members (18,20) comprising a plurality of plates (19,21), each plate (19,21) comprising a plurality of slits (22,26),
- a supporting frame (30) encasing said grid structure (12), said supporting frame (30) comprising a first set of frame elements (32) forming two opposite longitudinal walls (32) of the supporting frame (30) and a second set of frame elements (34) forming two opposite transversal walls of the supporting frame;
- a mesh substrate (40) arranged on said upper surface of the grid structure (12),
and
- an insert (48) arranged on the upper surface of the mesh substrate (40), wherein the insert (48) is coupled to the grid structure (12) via movable fixation means (50).

## Description

### TECHNICAL FIELD

The present invention relates to the field of absorbent articles and a method to form the absorbent core of said absorbent article. More precisely the present invention pertains to a forming pocket for forming absorbent core comprising at least one channel substantially free of absorbent material for absorbent articles, the forming pocket comprising an external forming substrate, suitable for receiving absorbent material and forming conglomerates from the fibrous or particulate materials, and a grid supporting structure that can be coupled with the external forming substrate. The present disclosure further relates to an apparatus for forming moulded absorbent material deposit structures to be used as absorbent core for absorbent articles. The present disclosure further relates to a method of making forming pocket. The present disclosure further relates to a process for providing at least two absorbent cores with different channel designs, for an absorbent article. The present invention is advantageously used in a forming drum conveyor of absorbent articles comprising a plurality of forming pockets that are suitable for forming respective absorbent core for absorbent articles in an apparatus for forming said absorbent articles, to which reference will be made below without loss of generality.

### BACKGROUND

As known, disposable absorbent articles such as diapers or training pants for babies, sanitary napkins or towels or products for adult incontinence, comprises an absorbent core arranged in between a liquid permeable topsheet and a liquid impermeable backsheet. The absorbent core comprises absorbent material such as cellulosic fibres or fluff or fluff pulp and/or of particles of superabsorbent material (SAP). The absorbent material is usually contained, encapsulated or enclosed in a core wrap. The core wrap can comprise one core wrap sheet extending above and under and on the side of the absorbent material thereby fully or at least partially covering the absorbent material. Alternatively, the core wrap can comprise two core wrap sheet, an upper core wrap and a lower absorbent core wrap, the absorbent material between arranged in between. In recent years, more and more absorbent cores comprise at least one channel substantially free of absorbent material. The at least one channel is formed by adhering the lower core wrap sheet to the upper core wrap sheet, or the portion of the sheet above to the portion of the sheet under when the core wrap comprises one single sheet. Preferably less than 1 wt.%, of superabsorbent polymer is present in said channel(s). In other terms, by "channels", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5 mm to 50 mm, more preferably from 8 mm to 40 mm, more preferably from 10 mm to 30 mm, even more preferably from greater than 10 mm to less than 25 mm.

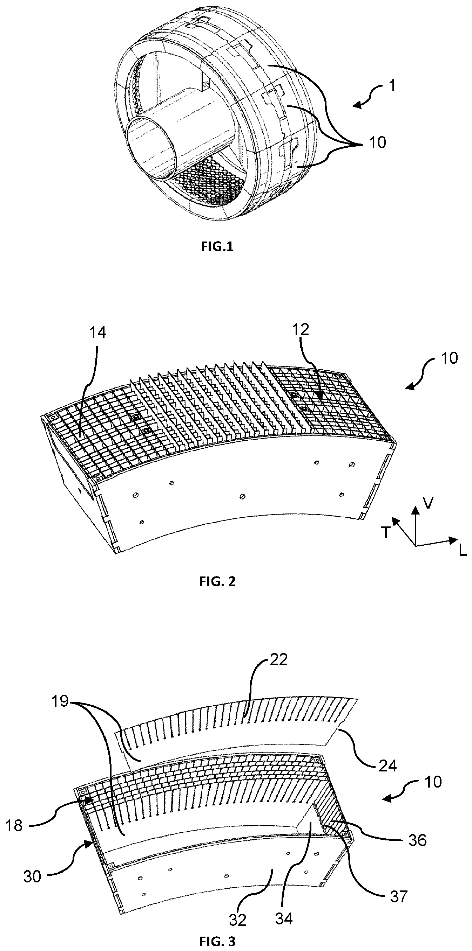

The forming apparatus (which is not illustrated) comprises a forming drum 1 (FIG. 1) of absorbent cores, the outer periphery of which is provided with a plurality of forming pockets 10 enabling the circulation of air, and is supplied, at the periphery thereof, with a non-woven material and/or a flow of absorbent material such as superabsorbent polymer and/or fluff. In each forming pocket 10, the non-woven and/or absorbent materials are maintained in the forming pocket 10 and conveyed from one point to another by a flow of sucking air and are expelled from the forming pocket 10 by a flow of blowing air.

As shown in FIG. 1, the forming drum 1 comprises a plurality of forming pockets 10 shaped, aligned and uniformly distributed along the external surface of the drum 1, and eventually comprising a cavity. Each forming pocket 10 has the desired shape for the absorbent core made of non-woven and absorbent material to be obtained and/or to permit the successive processes for which the absorbent material conglomerate is intended. The depth of the forming pocket determines the thickness of the absorbent core to be made. The forming pockets 10 are typically fixed to spaces of the forming drum 1 of a shape corresponding to the pockets.

The forming pockets 10 are perforated to enable the air flow to retain effectively by suction the absorbent material and eventually non-woven material on the surface thereof, but at the same time they prevent said absorbent material and/or non-woven from traversing the forming pockets 10.

Publications US 6,630,096 or US 2013/0139,960 illustrate example of forming pockets using dedicated shapes, but as explained in publication US 10,603,222, the method to produce these forming pockets requires dedicated and expensive equipment. Special welding work benches are in fact necessary, on dedicated electro-erosion machining work benches, which are very expensive. The electro-erosion process is in itself very expensive because it requires dedicated electrodes that are to be regenerated frequently inasmuch as they are subject to wear. In addition to the dedicated equipment, several machining steps are necessary, which make the production process of each grid supporting structure lengthy and which further require much labour. It is added that the shape of each grid supporting structure is determined by the shape of the corresponding absorbent padding to be made and that accordingly the equipment dedicated to the production of a specific type of supporting structure has to be modified with the variation of the type of absorbent padding to be made. Rather publication US 10,603,222 shows how by wedging comb-shaped plates altogether, a cheaper alternative can produce efficient forming pocket. While addressing the cost issues and finding a cheaper alternative, publication US 10,603,222 does not offer any suggestion to form absorbent core with channels and how to adapt the system to produce absorbent cores of different sizes or to produce absorbent cores having channels of different shape and/or size.

It is thus an object of the invention to resolve this and offer an apparatus and method to form absorbent cores comprising channels, said method and apparatus being flexible and enabling an easy and secure way to produce absorbent cores comprising channels of different shape and/or size thereby guaranteeing a proper standardisation of the absorbent core forming process. In other words, the present disclosure describes an apparatus and method enabling a manufacturer producing absorbent cores with channels to switch easily from one channel design to another channel design.

### SUMMARY

The present disclosure pertains to a forming pocket, suitable for forming moulded absorbent material deposit structures to be used as absorbent core for absorbent articles, the forming pocket, extending in a longitudinal, transversal and vertical direction, comprises:
- a grid structure comprising a first set of grid members and a second set of grid members, each set of grid members comprising a plurality of plates, each plate comprising a plurality of slits,
   wherein the plates of the first set of grid members are configured to be wedged into the plates of the second set of grid members via said slits thereby forming said grid structure comprising an upper surface, a lower surface and four lateral surfaces, specifically two longitudinal and two transversal surfaces ;
   preferably said grid structure being configured to receive and support said mesh substrate, and comprising pores or openings to let air pass, of flow, through, preferably, each member of the first set and each member of the second set of the grid member has a respective external edge configured for defining part of an external lateral surface of the grid structure ;
- a supporting frame encasing, or carrying or supporting, at least partially, said grid structure, said supporting frame comprising a first set of frame elements forming two opposite longitudinal walls of the supporting frame and a second set of frame elements forming two opposite transversal walls of the supporting frame;
   preferably said supporting frame is encasing, or covering or enveloping, the lateral surfaces of the grid structure but not the upper and lower surfaces of the grid structure in order not to hinder the airflow passing through the forming pocket.
   wherein each longitudinal walls comprises a plurality of slots configured to receive the extremities of the plates of a first or second set of grid members and/or each transversal walls comprises a plurality of slots configured to receive the extremities of the plates of a second or first set of grid members ; for sake of clarity, if the longitudinal walls receive the extremities of the plates of the first set of grid members then the transversal walls receive the extremities of the plates of the second set of grid members and vice versa;
- a mesh substrate arranged on said upper surface of the grid structure, preferably said mesh substrate being configured to receive and support absorbent material and comprises pores, micro-pores or openings to let air pass through; and
- an insert arranged on said upper surface of the mesh substrate, in other words said insert is protruding from the surface of the mesh substrate on the side that is opposite the grid structure,
   wherein the insert is coupled to the grid structure via movable fixation means.

In this construction, with respect to the vertical direction of the forming pocket, the grid structure is arranged on the lower surface of the mesh substrate, or in other words, the mesh substrate is sandwiched between the insert and the grid structure. It is also possible according to an embodiment for a first or second set of grid members to be solidarized, i.e. forming continuity of matter, with the longitudinal or transversal walls, the other set of grid members being then wedged within the other walls of the supporting frame.

According to an embodiment, the movable fixation means are arranged on at least one plate of the first and/or second set of grid members.

According to an embodiment, the movable fixation means comprise a threaded fastening in which the insert comprises a threaded hole and the grid structure comprises a threaded screw or in which the insert comprises a threaded screw and the grid structure comprises a threaded nut or in which the insert and grid structure both comprise a threaded hole and a stud bolt threaded at each extremities said bolt engaging both insert and grid structure or in which the insert and grid structure both comprise a threaded screw with a stud bolt comprising threaded cavities at each extremities said bolt engaging both insert and grid structure.

According to an embodiment, at least one plate of the first and/or second set of grid members comprises at least one fixation flange comprising a through hole, said threaded screw or bolt passing through said through hole. Preferably, said fixation flange extends in said transversal and longitudinal directions.

According to an embodiment, at least one plate of the first and/or second set of grid members comprises at least one opening arranged in facing relationship to a fixation flange arranged on an adjacent plate, said fixation flange passing through said opening.

According to an embodiment, the movable fixation means comprise screws with U-shaped flanges, said plate of the first and/or second set of grid members comprising a cut-out where the height of said plate at said cut-out is less than the maximum height of said plate, said height corresponding to the distance along the vertical direction from one edge to the opposite edge for said plate.

According to an embodiment, each frame elements of the first set of frame elements comprises a four walls defining a tube with two longitudinal walls and the second set of frame element defines two transversal walls.

According to an embodiment, the forming pocket comprises a masking component arranged on the upper surface of the mesh substrate, said masking component being attached to the supporting frame and/or grid structure via additional fixation means. Preferably the masking component is of complementary shape as the absorbent core to be formed.

According to an embodiment, the mesh substrate comprises a portion extending partially along at least one transversal wall of the supporting frame, said supporting frame comprising a recessed area to receive said portion and/or a closing mean to secure said portion to said transversal wall.

According to an embodiment, each plate of the first set of grid member comprises a rectangular end-notch arranged at each extremity of said longitudinal plate, and each plate of the second set of grid member comprises a rectangular end-notch arranged at each extremity of said transversal plate, both longitudinal walls and both transversal walls comprising slots to accommodate said extremities of each plate respectively, said slots being arranged at different longitudinal or transversal positions and each slots partially extending along the vertical direction from one edge of said longitudinal wall or said transversal wall toward the opposite edge of said longitudinal or transversal wall. Preferably said slots are straight and extend between 20 % and 80 % of the total height of the corresponding frame members.

The present disclosure also pertains to an apparatus for forming moulded absorbent material deposit structures to be used as absorbent core for absorbent articles, said apparatus comprising:
- a forming drum comprising at least one forming pocket as described hereabove, said forming pocket extending in a longitudinal, transversal and vertical direction, the forming pocket being curved along the longitudinal direction and being adapted to be fixed onto the circumference of said drum; and
- a feeding device for feeding absorbent material within the forming pocket.

The present disclosure also relates to a method of making a forming pocket as described hereabove, wherein the method comprises the following steps :
1. providing a first and second set of frame elements and assembling them to define a supporting frame; and either
2a. providing a plurality of plates of the first set of grid member and wedging them within the supporting frame and providing a plurality of plates of the second set of grid member and wedging them within the supporting frame and plates of the first set of grid member;
2b. providing a plurality of plates of the second set of grid member and wedging them within the supporting frame and providing a plurality of plates of the first set of grid member and wedging them within the supporting frame and plates of the second set of grid member; or
2c. providing a plurality of plates of the first and second set of grid member and wedging them together to form a grid structure and wedging said grid structure within the supporting frame;
   and
3. providing a mesh substrate and fixing it to the grid structure and/or supporting frame; and
4. providing an insert and fixing it to said grid structure via movable fixation means.

According to an embodiment, the method comprises a further step of providing a masking component and fixing said masking component to the grid structure and/or supporting frame, this step being prior to or after step 4 mentioned hereabove.

The present disclosure also pertains to a process for providing at least two absorbent cores with different channel designs, for an absorbent article, the process comprising the steps of:
i. providing a forming pocket as described hereabove, said forming pocket defining a mould cavity ;
ii. arranging a first insert into the mould cavity and engaging said first insert with the grid structure via the movable fixation means;
iii. optionally placing a non-woven within the mould cavity;
iv. inserting one or more absorbent materials into the mould cavity comprising the first insert to form a first moulded absorbent material deposit structure;
v. optionally placing a second non-woven within the mould cavity on top of the absorbent materials;
vi. removing the first moulded absorbent material deposit structure from the mould cavity to form a first absorbent core comprising one or more first zones comprising a first amount of absorbent material and one or more second zones comprising a second amount of absorbent material, wherein said first and second amounts of absorbent material, each measured as a weight per unit area, are different;
vii. removing the first insert from said mould cavity;
viii. optionally removing the mesh substrate and changing the position of some plates comprising at least one fixation flange and/or cut-out;
ix. arranging a second insert into the mould cavity and engaging said insert with the grid structure via movable fixation means ;
x. optionally placing a non-woven within the mould cavity;
xi. inserting one or more absorbent materials into the mould cavity comprising the second insert to form a second moulded absorbent material deposit structure;
xii. optionally placing a second non-woven within the mould cavity on top of the absorbent materials;
xiii. removing the second moulded absorbent material deposit structure from the mould cavity to form a second absorbent core comprising one or more first zones comprising a first amount of absorbent material and one or more second zones comprising a second amount of absorbent material, wherein said first and second amounts of absorbent material, each measured as a weight per unit area, are different;
   wherein the first and second inserts are different in shape and/or dimensions.

### BRIEF DESCRIPTION OF THE DRAWINGS

The drawings and figures are illustrative in nature and not intended to limit the subject matter defined by the claims. The following detailed description can be understood when read in conjunction with the following drawings, where like structure is indicated with like reference numerals in which:
FIG. 1 illustrates a perspective view of a forming drum of absorbent cores for disposable absorbent articles ;
FIG. 2 shows a perspective view of a portion of a forming pocket according to an embodiment, specifically the grid structure and the supporting frame, assembled ;
FIG. 3 depicts a perspective view of a portion of the forming pocket of FIG. 2, specifically of the grid structure and the supporting frame, being assembled, namely with the first set of grid members only being arranged within the supporting frame ;
FIG. 4 represents a perspective view of a portion of the forming pocket, specifically of the grid structure and the supporting frame, being assembled, namely with the first and second sets of grid members being arranged within the supporting frame ;
FIG. 5 illustrates a perspective view of a portion of the forming pocket, specifically of the grid structure, the supporting frame and the mesh substrate, assembled ;
FIG. 6 shows a perspective view of a portion of the forming pocket, specifically of the grid structure, the supporting frame, the mesh substrate and the insert, assembled ;
FIG. 7A and FIG. 7B depict an example of fixation means for fixing the insert onto the forming pocket 10 ;
FIG. 8 represents a cross-section of a portion of the forming pocket comprising an insert ;
FIG. 9A and FIG. 9B illustrate another embodiment of fixation means for fixing the insert onto the forming pocket 10 ;
FIG. 10 shows a perspective view of a portion of a forming pocket according to another embodiment, specifically the supporting frame, disassembled ;
FIG. 11 represents a perspective view of a portion of the forming pocket of FIG. 10, specifically of the grid structure and the supporting frame, being assembled, namely with the first and second sets of grid members being arranged within the supporting frame ;
FIG. 12 depicts a perspective view of a portion of the forming pocket of FIG. 10, specifically of the grid structure, the supporting frame, the mesh substrate and the masking component, assembled ;
FIG. 13 shows a perspective view of a portion of the forming pocket of FIG. 10, specifically of the grid structure, the supporting frame, the mesh substrate, masking component and the insert with the insert being assembled onto the forming pocket;
FIG. 14 illustrates a perspective view of an assembled forming pocket of FIG. 10, specifically of the grid structure, the supporting frame, the mesh substrate, masking component and the insert with the insert being assembled onto the forming pocket
FIG. 15A represents a transversal cross section of the assembled forming pocket of FIG. 14, and FIG. 15B depicts a close-up of the fixation means in the assembled forming pocket of FIG. 14 according to a longitudinal cross section view.

### DETAILED DESCRIPTION OF THE INVENTION

The present disclosure concerns an improved forming pocket, suitable for receiving absorbent material and forming conglomerates from said absorbent material to be used as an absorbent core for absorbent articles. The present disclosure also pertains to a forming apparatus for making an absorbent padding for hygienic products using such forming pocket. The present disclosure also relates to a method for assembling a forming pocket. Finally the present disclosure also relates to a method for manufacturing absorbing articles of two different channels.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In this description, identical elements that are common to the various illustrated embodiments have been indicated by the same numbering.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Absorbent article" refers to devices that absorb and contain liquid, and more specifically, refers to devices that are placed against or in proximity to the body of the wearer to absorb and contain the various exudates discharged from the body.

Absorbent articles include but are not limited to diapers, adult incontinence briefs, training pants, tampons, diaper holders and liners, pantiliners, sanitary napkins and the like, as well as surgical bandages and sponges. Disposable absorbent articles comprise a liquid pervious topsheet, a liquid impervious backsheet joined to the topsheet, and an absorbent core positioned and held between the topsheet and the backsheet. The absorbent article, or absorbent assembly, may also include other components, such as liquid wicking layers, liquid intake layers, liquid distribution layers, transfer layers, barrier layers, wrapping layers and the like, as well as combinations thereof.

"Absorbent material" as used herein refers to the material constituting the absorbent core of the absorbent article. Examples of commonly occurring absorbent materials are cellulosic fluff pulp, tissue layers, highly absorbent polymers or superabsorbent polymer particles (SAP), absorbent foam materials, absorbent nonwoven materials or the like. It is common to combine cellulosic fluff pulp with superabsorbent polymers in an absorbent material.

The term "channel(s)" as used herein means fluid distribution means within the absorbent core adapted to favor exudate flow there along and are typically intended to exclude embossing patterns or ducts formed by compression from the meaning thereof and rather include structures that are substantially free of absorbent material instead of comprising compacted absorbent material. Channels are commonly known in the art. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1 mm, preferably from 5 mm to 50 mm, more preferably from 6 mm to 40 mm, more preferably from 8 mm to 30 mm, even more preferably from greater than 8 mm to less than 25 mm. The one or more channels are arranged within the inner periphery of the absorbent core, i.e. the inner, or interior, space defined by the periphery of the absorbent core, such that each of the channel(s) is bounded, or surrounded, by absorbent material.

Additionally, the length of the channels may be from 10 % to 95 % of the length of the absorbent core so that again the one or more channels are bounded by absorbent material. By "channel design" it is meant a general design of said channel, e.g. an outline of the channel, or by extension an arrangement of the corresponding channel insert, with a given shape and/or dimensions or more broadly an underlying scheme that governs the function of ensuring a distribution of fluid.

"Comb-shaped cuts", "slots", "embrasures" and "slits" as used herein are synonymous and mean a long, narrow cuts or openings. For example a plurality of comb-shaped cuts in a plate means a plurality of long, narrow cuts or openings , preferably parallel, arranged within said plate.

"Comprise," "comprising," and "comprises" and "comprised of' as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "distinct" as used herein means separate, unconnected or discrete, i.e. not forming a continuity of matter.

The term "fixation means" encompasses the connection between two elements such as the grid structure and the insert, as well as any or all the different components of said connection, meaning any or component which play a role (e.g. fastening, supporting, maintaining, distributing loads,...) in this connection, such as a screws, cut-outs, fixation flanges, threaded nuts, threaded holes, U-shape flanges, screw sleeves, bolts, bolt studs, screw shanks, screw heads, threads, washer, pins, pegs, *etc.*

The term "grid" or "grid structure" as used herein means a framework or network of spaced bars or plates that are parallel to and/or cross each other; a grating or a network of elements, e.g. plates, that cross each other to form a series of squares or rectangles or diamond-shaped spaces. A grille, a grating or a lattice are synonyms of "grid". As used herein, a "grid structure" comprises interlaced structures such as lattices or networks of chevron-shaped plates assembled together or networks of chevron-shaped plates assembled with rectilinear plates. As used herein, a "grid structure" means a structure or network comprising separate, or distinct elements, or members, such as plates that are assembled, or wedged, together to form a grid with at least some, even all, of these plates that can be dissociated, or detached or separated, from the other plates. In other words, the grid structure in the present disclosure is an assembly of bars or plates that can be assembled as well as be disassembled, specifically, said plates can be interchanged or inverted.

The term "longitudinal", "transversal" and "vertical" as used herein are with respect to the forming pocket in an assembled state or in a disassembled or partially assembled state. The term "in the direction" as used herein means along that direction, *e*.*g*. in the longitudinal direction means along the longitudinal direction. A grid member or a frame member being described as extending in a given direction is in reference to the forming pocket preferably once assembled, for example a transversal plate extending in the transversal direction means that this plate is extending in the same transversal direction as defined for the forming pocket or for example a fixation mean such as a screw extending upward or downwards means that this screw is extending in the same vertical direction as defined for the forming pocket or for example an upper surface and a lower surface means two surfaces where the upper surface is above the lower surface with respect to the vertical direction as defined for the forming pocket or in other words the upper surface is on top of the lower surface. The term "lateral" means on the sides of a given element, e.g. the "lateral surfaces of the grid structure" means the fours surfaces forming the sides of the grid structure, the two other surfaces being the top and bottom surfaces, or upper and lower surfaces.

A "lateral surface" extends in a plane defined by the longitudinal and vertical directions or in a plane defined by the transversal and vertical directions whereas an "upper surface" or a "lower surface" extends in a plane defined by the longitudinal and transversal directions.

The term "mesh" or "mesh substrate" as used herein means a screen, a net, a material made of a network of wire(s) or thread(s), an interlaced structure or a weblike pattern or construction, all that have small openings to let air pass through while retaining, or blocking other material such as absorbent material, nonwoven material, or in other words that is air permeable. Incidentally, a mesh defines smaller holes, meaning holes with smaller surface areas, than a grid.

The term "movable" as used herein means that can be moved, that is mobile or shiftable or more simply that can easily change place or position or that can easily be replaced, repositioned, inverted, interchanged or exchanged. In other words, the movable fixation means are not stationary or irreversibly fixed, they can be moved around if desired.

The term "non-woven material" means a sheet material having a structure of individual fibers or threads which are interlaid, but not in a regular manner such as occurs with knitting or weaving processes. Nonwoven fabrics or webs have been formed from many processes such as, for example, meltblowing processes, spunbonding processes, and bonded carded web processes.

The term "wedge with" or "wedge into" or "wedge to" are synonymous and mean fixed in position, preferably by being forced into a narrow space or a plurality of narrow spaces.

The recitation of numerical ranges by endpoints includes all numbers and fractions subsumed within that range, as well as the recited endpoints.

In the following detailed description of the preferred embodiments, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

A forming apparatus (which is not illustrated) for making absorbent cores for disposable absorbent articles comprises a forming conveyor of absorbent cores. A forming drum 1 is illustrated in FIG. 1. The forming drum 1, corresponding to a conveyor mean, comprises at least one forming pocket 10. The forming pocket 10 is arranged and configured, meaning suitable, for receiving absorbent material, such as superabsorbent polymer or superabsorbent particles and/or fluff, fluff pulp or cellulosic fibers, and forming conglomerates from said absorbent material to be used as absorbent cores for disposable absorbent articles.

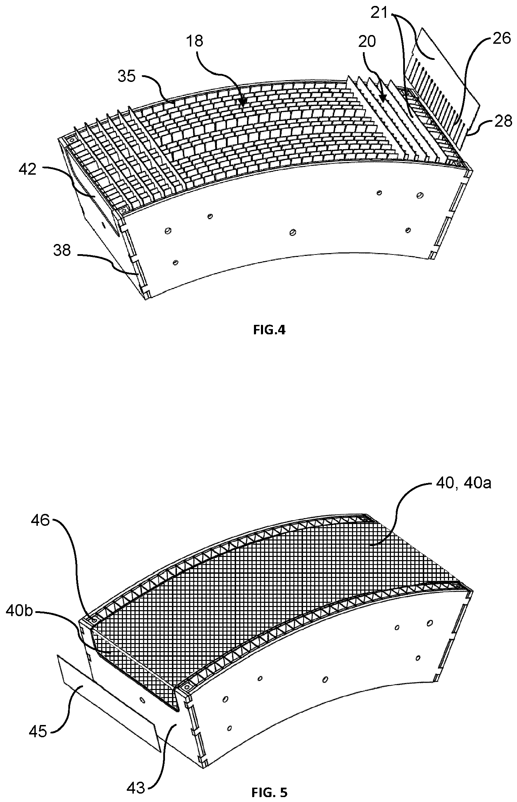

A semi-finished forming pocket 10 is illustrated in FIG. 2, meaning a part of a forming pocket 10 according to an embodiment is shown in FIG. 2. The forming pocket 10 comprises a grid structure 12 comprising, or rather defining, air-through openings 14 through which air can flow through. In other words, the grid structure 12 is air permeable. The forming pocket 10 also comprises a mesh substrate 40, illustrated in FIG. 5, with the grid structure 12 supporting, or carrying, said mesh substrate 40. The mesh substrate 40 is also air-permeable, i.e. configured to let air pass through. The absorbent material or eventually a non-woven material cannot not pass through the mesh substrate 40, hence the absorbent material and eventually non-woven material can be supported, carried or transported and maintained on or within the forming pocket 10, namely on the mesh substrate 40, by vacuum, or air suction with air generating a negative pressure to maintain said absorbent material onto the surface of the mesh substrate 40. The mesh substrate 40 is for example a perforated screen such as a wire mesh or a micro-perforated screen, preferably having a pore dimension which is less than the air permeable surface of the grid structure 12. It allows to hold the smaller material components of the absorbent core such as a nonwoven, e.g. a core wrap, or any absorbent material within the mould cavity defined by the forming pocket 10, avoiding that they become sucked by the vacuum applied through the porous, air permeable, bottom surface of the mould cavity, meaning the mesh substrate 40.

The grid structure 12 comprises a first set of grid members 18 and a second set of grid members 20, each set of grid members 18, 20 comprising an array of plates, preferably metallic plates, each comprising a plurality of comb-shaped cuts, embrasures or slits 22,26. The plates are preferably thin, flat sheet or strip made out of metal, for example said plates are preferably between 0.3 mm and 1.5 mm thick, preferably between 0.5 mm and 1 mm, for example 0.8 mm thick and are preferably made out of a resistant material such as stainless steel, hard anodized aluminium or titanium alloy, SAP being an abrasive material, it is best to use a resistant material. The grid members of the first set 18 and second set 20 are configured to be wedged together *via* said comb-shaped cuts thereby forming a grid of said grid structure 12. For example, the first set of grid members 18, as illustrated in FIG. 3, comprises an array of elongated plates 18 extending in the longitudinal direction of the forming pocket 10 whereas the second set of grid members 20, as illustrated in FIG. 4, comprises an array of elongated plates extending in the transversal direction of the forming pocket 10. Each plate comprises a plurality of slits 22,26 dimensioned to receive another plate so that all the different plates can be crammed together thereby forming a grid of made out of plates and defining openings.

As illustrated in FIG. 2, the forming pocket 10 extends in a longitudinal direction L (length), in a transversal direction T (width) and in a vertical direction V (height). Preferably, the forming pocket 10 comprises an average length, width and height where the length is greater than the width and the width is greater than the height. The forming pocket is curved, or arcuate, in the longitudinal direction L in order to match the outer diameter of the forming drum 1 and thus ensuring a better vacuum. The forming pocket 10 is rectilinear, or straight, in the transversal and vertical directions T,V.

As illustrated in FIG. 3, the first set of grid members 18 comprises elongated plates 19 extending in the longitudinal and vertical directions L,V of the forming pocket 10.

Each longitudinal plate 19 comprises a plurality of slits 22, or comb-shaped cuts or embrasures, extending in the vertical direction V. The slits 22 are arranged at different longitudinal positions of the longitudinal plate 19 and extend from one vertical extremity of the longitudinal plate 19 towards the opposite vertical extremity of the longitudinal plate 19. The slits 22 are preferably straight and extend between 20 % and 80 % of the total height of the longitudinal plate 19, preferably between 40 % and 60%, more preferably the height of each slit 22 equals to 50% of the total height of the longitudinal plate 19. Such construction enables a good fixation, or wedging, of the first set of grid member 18 into the second set of grid member 20.

As illustrated in FIG. 3, each longitudinal plate 19 also comprises a rectangular end-notch 24, or rectangular cut-out, arranged at each longitudinal extremities of said longitudinal plate 19. In other words, each longitudinal plate 19 comprises a slight bottle-neck longitudinal cross section. These end-notches 24 enable to better secure the grid members 18 within the forming pocket 10, namely within the supporting frame 30 that is described hereunder. In order to match the shape of the forming pocket 10, each grid member of the first set of grid member 18, meaning each longitudinal plate 19, is arcuate in the longitudinal direction L, or in other words, the axis linking the two notches 24 is curved.

As illustrated in FIG. 4, the second set of grid members 20 comprises elongated plate 21 extending in the transversal and vertical directions T,V of the forming pocket 10. Each transversal plate 21 comprises a plurality of slits 26, or comb-shaped cuts or embrasures, extending in the vertical direction. The slits 26 are arranged at different transversal positions of the transversal plate 21 extending from one vertical extremity of the transversal plate 21 towards the opposite vertical extremity of the transversal plate 21. The slits 26 are preferably straight and extend between 20 % and 80 % of the total height of the transversal plate 21, preferably between 40 % and 60%, more preferably the height of each slit equals to 50% of the total height of the transversal plate 21. Such construction enables a good fixation of the second set of grid member 20 into the first set of grid member 18.

As illustrated in FIG. 4, each transversal plate 21 comprises a rectangular end-notch 28, or rectangular cut-out, arranged at each transversal extremities of said transversal plate 21. These notches 28 enable to better secure the grid members 20 within the forming pocket 10, namely within the supporting frame 30. Each grid member of the second set of grid member 20, meaning each transversal plate 21, is rectilinear, or straight, in the transversal direction T, or in other words, the axis linking the two notches 28 is linear.

Both the first set of grid member 18 and the second set of grid member 20 comprise slits 22,26, i.e. both longitudinal plates 19 and transversal plates 21 comprises slits 22,26. These slits 22,26 enable a proper wedging, or cramming, of the first set of grid members 18 with, or into, the second set of grid members 20 and vice-versa. For this, the slits 22 of the first set of grid members 18 are arranged in the upper vertical portion of the longitudinal plates 19 and the slits 26 of the second set of grid members 20 are arranged in the lower vertical portion of the transversal plates 21. In other words, in a finished or assembled configuration, the slits 22 of the longitudinal plates 19 extend vertically with the indentation of the slits 22 being arranged at the vertical top of the longitudinal plate 19, and the slits 26 of the transversal plates 21 extend vertically with the indentation of the slits 26 being arranged at the vertical bottom of the transversal plate 21. The slits 22, 26 enable an interlocking of set of grid members 18,20, namely an interlocking of longitudinal plates 19 with transversal plates 21, thereby defining a grid structure 12 where each longitudinal plate 19 is wedged with multiple transversal plates 21 and each transversal plate 21 is wedged with multiple longitudinal plates 19. The slits 22,26 are preferably rectilinear for an easier wedging. As illustrated in FIG. 3, the first set of grid members 18 is first placed within the forming pocket 10, namely within the supporting frame 30, then the second set of grid members 20 is wedged into the first set of grid members 18 thereby forming a grid structure 12.

In other words, the slits or cuts 22,26 of each longitudinal or transversal plates 19,21 of the first set extend from the vertical edge to the opposite vertical edge without intersecting said opposite vertical edge. The grid members 20 of the second set are suitable for wedging in the grid members 18 of the first set to make the grid of the grid structure 12. In this manner, each transversal plates 21 of the second set of grid member 20 can be wedged into a plurality of longitudinal plates 19 of the first set of grid member 18, i.e. by placing the indentations of each slits 26 of the transversal plates 21 above the indentations of each slits 22 of the longitudinal plates 19 and by pushing, or sliding, the transversal plates 21 downward.

Naturally, the invention is not limited to this arrangement particularly. For instance the slits 22,26 can be vertically inverted, meaning that the slits 22 of the first set of grid members 18 are arranged in the vertical lower end of each longitudinal plate 19 whereas the slits 26 of the second set of grid members 20 are arranged in the vertical upper end of each transversal plate 21. In this arrangement, the second set of grid members 20 is first placed within the forming pocket 10, namely within the supporting frame 30, then the first set of grid members 18 is then wedged into the second set of grid members 20 thereby forming a grid structure 12.

According to alternative embodiments, the slits 22,26 can be arranged within their respective longitudinal or transversal plate 18,20 in regular intervals to have a regular grid pattern or the slits 22,26 can be arranged in irregular intervals to have an irregular grid pattern.

According to another embodiment, the slits 22 of the first of grid members 18 are arranged in regular intervals whereas the slits 26 of the second grid members 20 are arranged in irregular intervals or vice-versa.

According to an embodiment, the slits 22,26 of the first and/or second set of grid members 18,20 comprise, or are arranged with, gaps 60 in between with slits 22,26 being arranged at regular interval with one or more gaps 60 being arranged between two slits 22,26.

Naturally, the present disclosure is not limited to these embodiment and it is possible to combine these embodiments, for example, the slits 22,26 can be vertically inverted as described above and the slits can be arranged with gaps 60 in between some slits 22,26 as described above.

The forming pocket 10 further comprises a supporting frame 30 to partially encase and carry the first and second set of grid members 18,20. In other words, the supporting frame 30 surrounds laterally, or surrounds longitudinally and transversally, the longitudinal and transversal plates 19,21. The supporting frame 30 comprises a first set of frame members 32 comprising two opposite longitudinal walls 32 of the supporting frame 30 and a second set of frame elements 34 comprising two opposite lateral walls, or two opposite transversal walls 34 of the supporting frame 30. One or both longitudinal walls 32 and/or one or both transversal walls 34 preferably comprises slits 35,36 to accommodate, or receive, the extremities of each set of first and second grid members 18,20 respectively. Specifically, the first set of frame members 32, i.e. the longitudinal walls 32, receive the transversal extremities of each second grid members 20, meaning of each transversal plates 21, and the second set of elements 34, i.e. the transversal walls 34, receive the longitudinal extremities of each first grid members 18, meaning of each longitudinal plates 19. In other terms, each frame member of the first set 32 and each frame member of the second set 34 has a respective internal edge configured for defining a part of the external transversal and longitudinal surface of the grid structure 12, a respective external edge opposite said respective internal edge, and side edges. In otherwords, each longitudinal wall 32 comprises a plurality of slots 35 configured to receive the protruding ends, e.g. the notches 28, of the side edges of the transversal plates 21 and each transversal wall 34 comprises a plurality of slots 36 configured to receive the corresponding protruding ends, e.g. the notches 24, of the side edges of the longitudinal plates 19. The forming pocket 10 comprises a base which is the grid structure 12 and/or the supporting frame 30 which is/are adapted to be fixed onto the circumference of the forming drum 1.

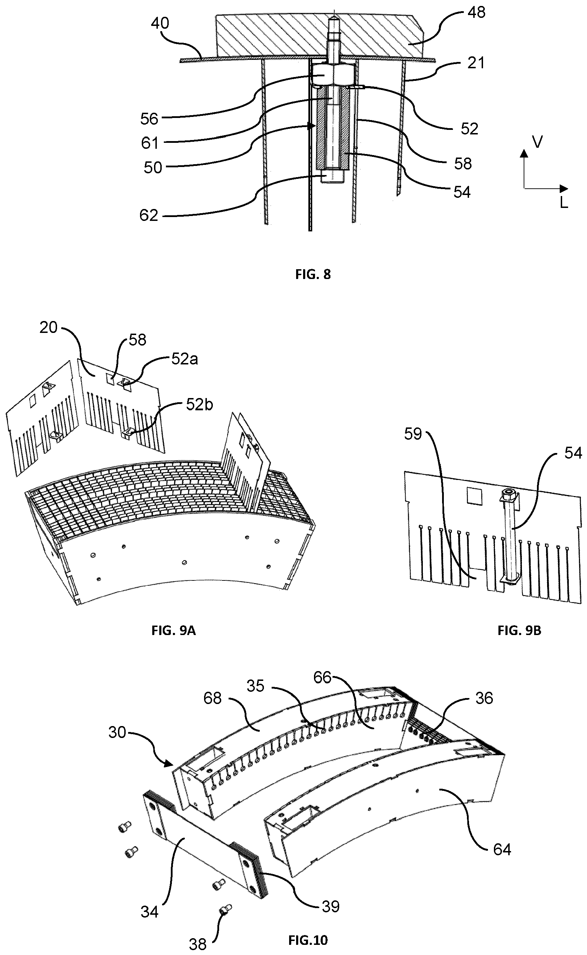

The invention is not limited to a single wall, for example, the first set of frame member 32 can comprise a hollow beam structure as illustrated in FIG. 10 according to an embodiment described hereunder.

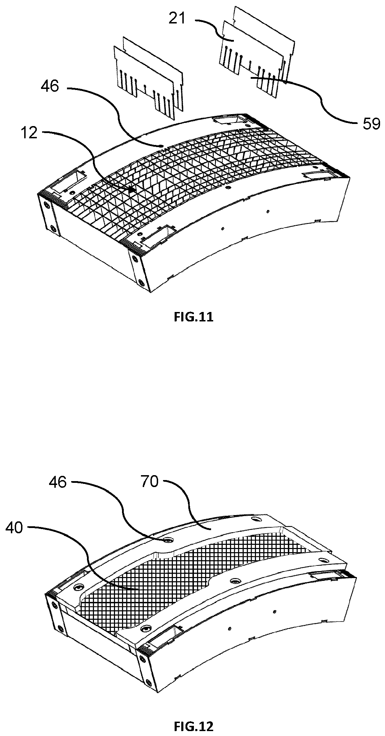

The slits 35,36 are arranged at different transversal or longitudinal positions extending from one vertical extremity of the first and second set of frame members 32,34 towards the opposite vertical extremity of said longitudinal or transversal walls 32,34. The slits 35,36 are preferably straight and extending between 20 % and 80 % of the total height of the corresponding frame members 32,34 on which the slit(s) is arranged, preferably between 40 % and 60 %, more preferably the height of each slit equals to 50 % of the total height of the corresponding frame member 32,34. Each longitudinal and transversal wall 32,34 also comprises a notch 37 of complementary shape to the extremities of the longitudinal or transversal plate 19,21. In other words, each grid member 18, 20 is configured to slide or be wedged into the slots 35,36 arranged in the frame members 32,34 thereby forming a consolidated grid structure 12 as illustrated in FIG. 2 or 11. The grid structure 12 defines a plurality of openings 14 each defined by four intersecting plates, two longitudinal plates 19 and two transversal plates 21.

According to an embodiment, the slits 35 in the first set of frame members 32 are arranged in regular intervals whereas the slits 36 in the second set of frame members 34 are arranged in irregular intervals or vice-versa.

According to an embodiment the slits 35,36 of the first and/or second set of frame members 32,34 can comprise gaps 60 where the slits 35,36 are arranged in regular interval with one or more gaps 60 being arranged between two slits 35,36.

According to an embodiment, the first and second set of frame members 32 comprises two distinct longitudinal walls 32 and two distinct transversal walls 34. Alternatively, the frame structure 30 can comprise one single piece where the first and second set of frame member 32 form a continuity of matter. It is also possible for the frame structure 30 to comprise two frame pieces, e.g. two longitudinal walls and a transversal wall forming a continuity of matter in a U-shape arrangement and a distinct transversal wall closing said U-arrangement for form the frame structure 30.

When the frame structure 30 comprises several, i.e. at least two, discrete members, the first set of frame members 32 and/or second set of frame members 34 preferably comprise interlocking means 38 to fasten the longitudinal walls 32 and transversal walls 34 together and define, or assemble, a frame structure 30 enclosing, or encasing, the grid structure 12 longitudinally and transversally. The interlocking means 38 are selected from and not limited to screws and threads (FIG. 10), adhesive, pins, pegs, matching or complementary shapes such as corresponding merlons and crenels (FIG. 4), box joints, bridle joints, dovetail joints, butt joints, protruding flanges. In other terms, each longitudinal wall 32 is fixed to two transversal walls 34, one at each side end; and each transversal wall 34 is fixed to two longitudinal walls 32, one at each side end. This fixation is preferably reversible, meaning that a longitudinal wall 32 can be detached, or separated, from a transversal walls 34 without destruction of matter, or irreversible damage.

The forming pocket 10 further comprises a mesh substrate 40 configured to receive, maintain, support and transport absorbent material and/or non-woven material. The mesh substrate 40 comprises small openings to let air pass through while retaining the absorbent material and/or non-woven material. The mesh substrate 40 is arranged on the grid structure 12, meaning the mesh substrate 40 extends longitudinally and transversally on top of the grid structure 12. In a view from above, i.e. top-view, the total surface of the mesh substrate 40 can be greater or equal to the upper surface of the grid structure 12 or the inner surface defined by the frame 30, e.g. as illustrated in the embodiment of FIG. 12. Alternatively, the surface of the mesh substrate 40 can be lesser than the surface of the grid structure 12, e.g. as illustrated in the embodiment of FIG. 5. Preferably, when seeing the forming pocket 10 from a top view, the total surface of the mesh substrate 40 is comprised between 80% and 100 % of the total surface of the grid surface 12, for example between 80% and 99 %.

The mesh substrate 40 may optionally further comprise a portion 40b which extends partially along one or both transversal walls 34, meaning that the mesh substrate comprises a main portion 40a extending over the grid structure 12 and a minor portion 40b extending along one or both transversal wall 34. As illustrated in FIG. 5, e.g. the forming pocket 10 can comprise a recessed area 42 dedicated to receive said minor portion 40b of mesh substrate 40. The recessed area 42 is arranged on the upper portion of the transversal wall 34 and can correspond to an area of the transversal wall 34 that is thinner. The recessed area 42 has the same width as, or a slightly larger width than, the mesh substrate 40 and extends vertically from 1 % to 50 %, preferably from 5 % to 25 %, of the total height of the transversal wall 34. In other words, the recessed area 42 is dimensioned and arranged to receive said portion 40b of the mesh substrate 40. Preferably the mesh substrate 40 is arranged within the recessed area 42, this arrangement enables to better secure the mesh substrate 40 onto the forming pocket 10 and supporting frame 30. The minor portion 40b of the mesh substrate 40 can also extend along a portion of a transversal wall 34 that has no recessed area 42.

In order to secure the mesh substrate 40 to the transversal wall 34, preferably within the recessed area 42, the forming pocket 10 can comprise a closing mean 45. The closing mean 45 can be selected from and not limited to a fastening tape, a cover with fastening means e.g. snap fit configured to interlock said cover with the transversal wall 34, one or more grooves where the mesh substrate 40, or the cover 45, can slid into, pins, screws, adhesive. The mesh substrate 40 extends partially or completely over the top surface of the grid structure 12, main portion 40a, and extends partially over the surface of one or both transversal wall 34, preferably within a recessed area 42 arranged on one or both transversal walls 34, minor portion 40b. This construction enables a good fastening of the mesh substrate 40 onto the grid structure 12 and frame structure 30 and within the forming pocket 10. According to an embodiment, the forming pocket 10 does not comprise any recessed area 42, the mesh substrate 40 extending along the transversal wall is fixed to this wall with a fastening tape 45, e.g. adhesive tape.

As illustrated in FIG. 5 and FIG. 12, the forming pocket 10 can also comprise additional fixation means 46. These additional fixation means 46 enable the fixation of a masking component 70. The masking component 70 may cover a portion of the grid structure 12 and/or frame 30 and/or mesh substrate 40 and defines the periphery of the absorbent core thereby defining its' overall shape or outer contour. The additional fixation means 70 can be selected from and not limited to screws and threads (FIG. 12), adhesive, pins, pegs, matching or complementary shapes. The mesh substrate 40 can be fixed, more precisely clamped, in between the masking component 70 and the frame structure 30.

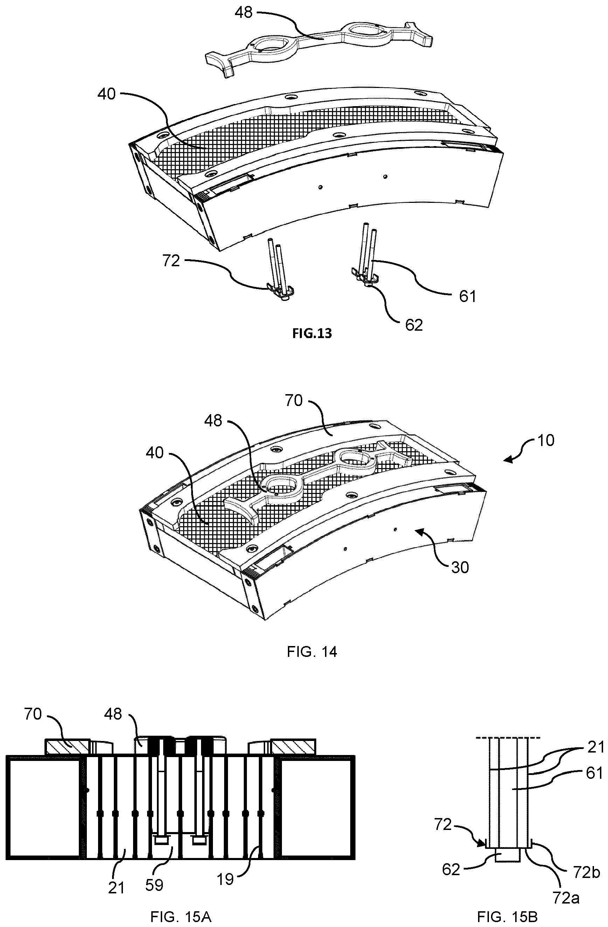

The forming pocket 10 also comprises an insert 48 arranged on a side of the mesh substrate 40, namely on the upper side of the mesh substrate 40. In other words the mesh substrate 40 is arranged in-between the insert 48 and the grid structure 12. It can also be said that the frame 30 and the grid structure 12 support the mesh substrate 40 the latter supporting the insert 48. The insert 48 is a mould piece or a shaping profile member that enables to defines channels within the absorbent core. In other words, the insert 48 is a three-dimensional structure inserted into the forming pocket 10 prior to depositing one or more absorbent materials into forming pocket 10. The insert 48, or channel insert 48, protrudes from the mesh substrate 40 surface (FIG. 8 or FIG. 15) and defines a region with little or no absorbent material. In other words, the combination of a masking component 70, a channel insert 48 and the mesh substrate 40 form a mould cavity, i.e. the masking component 70 and mesh substrate 40, comprising a shaping profile member, i.e. the channel insert 48, to form an absorbent core with a given outer contour and channels. The insert 48, or shaping profile member or three-dimensional structure may be configured in any desired shape to provide zones or lines of absorbent material having a density which is lower than the density of the one or more first zones or lines of absorbent material, *e*.*g*. the bulk density of the bulk absorbent material.

The insert 48 is coupled to the grid structure 12 via movable fixation means 50. In particular, the insert 48 is reversibly fastened to the grid structure 12 via these fixation means 50 that can be moved. By the term "reversibly fastened" as used herein, it is meant that the insert 48 is connected in a reversible manner to the grid structure 12. In other words the forming pocket 10 comprises three components, elements or modules, specifically the insert 48, the mesh substrate 40 and the grid structure 12, that are discrete, or separate, meaning that these three components do not form a continuity of matter. These components are associated, or engaged, with one another in a reversible manner, meaning that they can be associated and separated without causing any irreversible damage. Preferably, the components are mechanically attached and separable without causing any irreversible damage.

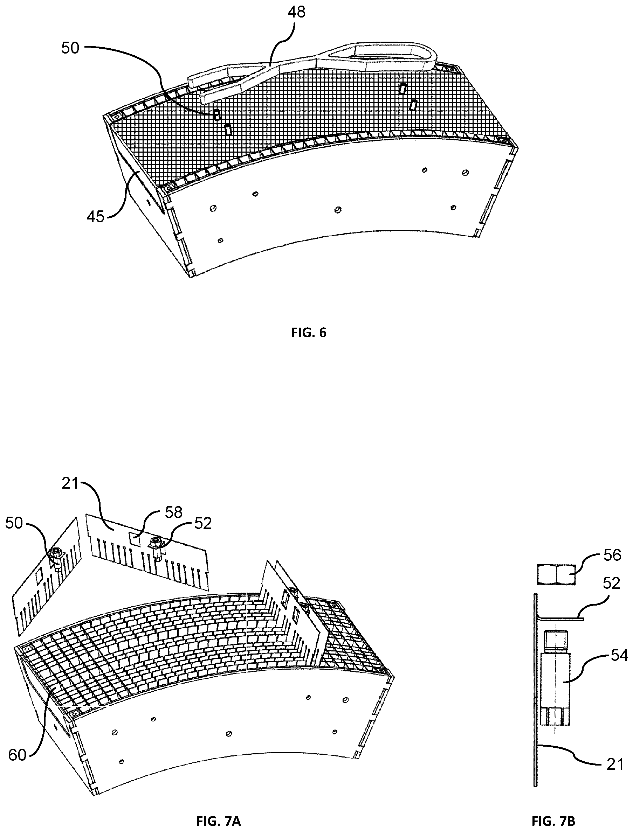

Such attachment enables to easily change or remove the insert 48 and thus simplifies the process in term of standardization. For example it is easy to change from an insert 48 having a shape as illustrated in FIG. 6 to an insert 48 having a shape as illustrated in FIG. 14, or to remove the insert 48 to make channel-less absorbent cores. The fixation means 50 are movable, or in other words they can be moved, and enable a reversible fixation between the insert 48, mesh substrate 40 and grid structure 12, though it may be necessary to make new holes within the mesh substrate 40, and if a mesh substrate 40 has too many holes, it can be easily replaced. The fixation means 50 can be selected from and not limited to screws and threads, bolts and nuts, pins, pegs, bayonet mount, twist-lock mount, matching or complementary shapes. Preferably, the fixation means 50 comprises a bolted joint. The mesh substrate 40 can comprise though-holes for the passageway of the fixation means 50 between the insert 48 and the grid structure 12. The fixation means 50 preferably comprises one, two, three or more components to enable the fixation of the insert to the grid structure. For example, the fixation means 50 comprise a male threaded fastener arranged within the grid structure 12 which is secured with a matching female screw thread arranged within the insert 48, the fastener and the screw thread being of complementary shape to ensure a proper fastening. Naturally, the invention is not limited to the number of fixation means 50 or components and the fixation means 50 can comprise one, two, three, four, five, six or more screws and corresponding threads.

The FIG. 7A and 7B illustrate an embodiment of the forming pocket 10 with fixation means 50. Here at least one transversal plates 21, preferably two, three or four transversal plates 21, comprise at least one fixation flange 52, or in other words at least one tab, each protruding from a transversal wall of said transversal plate 21. The fixation flange 52 extends in a plane defined by the longitudinal and transversal directions and comprise a through-hole. The fixation flange 52 serves as support to a fixation mean 50 meaning to one or more components of a fixation mean 50, e.g. screw 61, screw head 62, screw sleeve 54 or threaded nut 56. The movable fixation means 50 comprises a screw 61, or a male fastener, or a bolt which is arranged on the grid structure 12, the fixation flange supporting or carrying said male component of the fixation means 50. The complementary, or corresponding, fixation mean 50, e.g. the female screw thread or threaded hole, is arranged on the insert 48, the screw 61 connecting and associating, or joining and maintaining under tension, the fixation mean 50, meaning the components of this fixation mean 50, altogether. As illustrated in FIG. 6, the channel insert 48 comprises a plurality of fixation means 50, here the channel insert 48 comprises four fixation means 50 being arranged two by two at each longitudinal ends of the insert 48, *e*.*g*. at the same longitudinal position of the forming pocket 10 and transversally separated. As illustrated in FIG. 7B and 9A, a fixation flange 52 comprises a plate extending perpendicular from the transversal plate 21 and comprising a through hole to enable a fastener, such as a screw, to pass through said through hole, or clearance hole. In other words, the fixation flange 52 or tab defines with said plate 21 a frame with an inner void, *i*.*e*. the through hole, through which the fastener can pass through. The fixation flange 52 is sandwiched between the threaded nut 56 and the sleeve 54 in an assembled state. By unscrewing the screw 61 from the threaded hole arranged in the insert 48, it is possible to dissociate the insert from the grid structure 12 and mesh substrate 40 and to remove said insert 48. When a threaded hole is arranged in the insert 48, said hole is preferably a blind hole as illustrated in FIG. 6 and FIG. 8 to avoid absorbent material entering the upper portion of the insert.

The embodiment as illustrated in FIG. 7A, 7B exemplifies such fixation means 50 and connection between insert 48 and grid structure 12. In this embodiment, two transversal plates 21, successive in the longitudinal direction L, each comprise a fixation flange 52 to support a fixation mean 50. The two fixation flanges 52 are extending in facing relation where the fixation flange 52 on one transversal plate 21 extends towards the successive, or adjacent, transversal plate 21 comprising the other fixation flange 52. The two fixation flanges 52 are at a distance in the transversal direction T. Each said transversal plate 21 comprises an opening 58 to receive the fixation flange 52 protruding from the other transversal plate 21 as illustrated in FIG. 7A or FIG. 8. The grid structure 12, specifically the first set of grid members 18 comprises a gap 60 where the longitudinal plates 19 are spaced apart at regular intervals, with two gaps 60, or two intervals greater than the regular interval, being arranged in the central region with respect to the transversal direction T. Specifically, as illustrated in FIG. 7A, the first set of grid members 18 comprises with respect to the transversal direction, six longitudinal plates 19 spaced apart at a uniform or regular interval, a gap 60 or greater interval, three longitudinal plates 19 also spaced apart at the same uniform or regular interval, another gap 60 and six longitudinal plates 19 also spaced apart at the same uniform or regular interval. The fixation mean 50 can be arranged within said gap 60. The second set of grid members 20, i.e. the transversal plates 21, do not comprise any slot 26 within these gap 60 regions. In other words, two consecutive or successive, with respect to the longitudinal direction, transversal plates 21 form a pair, each said plate 21 comprising an opening 58 and a protruding flange 52 serving as support to a fixation mean 50; the opening 58 and protruding flange 52 being arranged in a facing, or mirror, arrangement where the opening 58 on one said transversal plate 21 faces the protruding flange 52 on the other said transversal plate 21 and vice versa. The protruding flanges 52 and openings 58 are preferably arranged on the upper portion of the transversal plates 21, with respect to vertical direction V. The grid structure 12 comprises here a second pair of transversal plates 21 each comprising an opening 58 and a protruding flange 52 supporting a fixation mean 50, thereby ensuring four fixation points for the insert 48 as illustrated in FIG. 6. The fixation of the insert 48 to the grid structure 12 will be further described hereunder. The present disclosure is not limited to this arrangement, for example a transversal plate 21 can comprise two fixation flange 52 and the consecutive, or adjacent or successive, transversal plate 21 can comprise the two corresponding openings 58.

With such arrangement, not only can the insert 48 be easily changed to a different shape or removed, it is also possible with this grid structure 12 and fixation means 50, to exchange inserts 48 of different sizes with different fixation points. Indeed, with this forming pocket, it is possible to remove the insert 48 by unscrewing the screw 61, remove the insert 48, remove the mesh substrate 40 by removing the closing means 45, and shift, or move with respect to the longitudinal direction L, the transversal plates 21 comprising the fixation means 50. The transversal plates 21 being reversibly, or removably, wedged into the first set of grid member 18, it is possible to pull out, or unwedge, some transversal plates 21 and change their longitudinal position in the grid structure 12. In other words, the grid structure comprises a plurality of plates 19,21 where at least some, preferably all, are reversibly wedged together meaning that the plates can be separated from one another to be interchanged, exchanged or inverted, preferably the plates 19,21 comprising the fixation flanges 52 and/or openings 58 and/or cut-outs 59 as are described hereunder, will be moved around and repositioned.

According to an embodiment as illustrated in FIG. 8, the fixation mean 50 comprises a screw 61 that is inserted from bottom to top, with respect to the vertical direction V and to the forming pocket 10 once assembled, and fixed to the insert 48 thereby associating the grid structure 12, mesh substrate 40 and insert 48 together. The insert 48 and nut 56 both comprise threaded holes, in which the rod of the screw 61, or stud, is screwed in until the head 62 of the screw abuts against the sleeve screw 54. Naturally, the disclosure is not limited to this embodiment, for example according to another embodiment not illustrated, the insert 48 comprises a threaded screw extending downward and is fixed to a nut 56 arranged on the lower end of the grid structure 12 with respect to the vertical direction, the protruding fixation flange 52 is also on the lower portion of the transversal plate 21 with respect to the vertical direction, the protruding fixation flange 52 being above the nut 56 with respect to the vertical direction.

Both FIG. 9A and FIG. 9B illustrate another embodiment of the fixation means 50. Here, at least one, preferably two, three orfour, transversal plates 21 comprises two fixation flanges 52a,52b that are vertically aligned and arranged at two opposite vertical end regions of the transversal plates 21. These transversal plates 21 comprises two corresponding openings 58 to receive the protruding flanges 52 arranged on an adjacent transversal plate 21, similarly as described above. These transversal plates 21 can comprise at least one cut-out 59 instead of an opening 58, meaning that the height of the transversal plate 21 is lesser than the maximum, or total, height of the transversal plates 21 as illustrated in FIG. 9B. The cut-out(s) 59 are preferably arranged between two slits 26 and in a central region of said transversal plate 21 with respect to the transversal direction. The screw sleeve 54 extends between the two fixation flanges 52 and the screw 61 is inserted from bottom to top, with respect to the vertical direction, and fixed until the head 62 abuts against the lower fixation flange. The lower fixation flange 52 serves as a washer arranged between the screw sleeve 54 and the screw head 62 and enables a better distribution of the load of the screw 61. The present disclosure is not limited to this arrangement, for example a transversal plate 21 can comprise four fixation flange 52 and the consecutive, or adjacent or successive, transversal plate 21 can comprise the four corresponding openings 58 and/or cut-outs 59, or for example three fixation flanges 52 on one transversal plate 21 and one fixation flange 52 on the transversal consecutive plate 21.

FIG. 10 to FIG. 15 illustrate another embodiment of the forming pocket 10. In this embodiment, the first set of frame element 32 comprises a tube, preferably rectangular, with four walls defining an interior volume. The tube is composed of an outer wall 64, defining the outer, or external, surface of the supporting frame 30 and thus the outer surface of the forming pocket 10, an inner wall 66 defining the inner surface of the supporting frame 30 and the outer surface of the grid structure 12 and two connecting plates 68 bridging, or linking, the inner wall 66 and the outer wall 64 thereby forming a rectangular tube. The first set of frame element 32 can comprise four discrete plates assembled together to form the rectangular tube, or the first set of frame element 32 can comprise two rectangular beams that can be hollow or not. In other words, each frame element of the first set of frame element 32 can be formed in one single piece forming a continuity of matter, e.g. a beam hollow or not, or in multiple plates assembled together to form a tube. These multiple plates can be assembled together through any conventional method such as welding, gluing, fit-forming, screwing, clipping. The inner wall 66 comprises slots 35 as described herein so that the second set of grid members 20, i.e. the array of transversal plates 21, can be fixed within the supporting frame 30. The slots 35 extends vertically on a portion of the inner wall 66. The second set of frame element 34 comprises two transversal walls 34 with slots 36 as described hereabove and as illustrated in FIG. 10. As illustrated in FIG. 10, the outer wall 64 is longer than the inner wall 66. Both longitudinal ends of each tube, i.e. of the first set of frame element 32, is fixed to, or assembled with, the second set of frame element 34. The tube and the second set of frame element 34, also comprises an interlocking means 38, here screw fasteners and threaded holes, to fasten the first set of elements 32 and second set of elements 34 together and define a frame structure 30, eventually with a washer 39 to distribute the load.

As shown in FIG. 11, the first set of grid member 18 and second set of grid member 20 are then introduced and wedged together within the frame structure 30 to define the grid structure 12 as previously described. The frame structure 30, and more specifically the connecting plate 68 arranged on top, or the nearest to the mesh substrate 40, comprise additional fixation means 46 to enable the fixation of a masking component 70 (FIG. 12) covering a portion of the grid structure 12 and optionally the mesh substrate 40 and defining the periphery or outer contour of the absorbent core. The mesh substrate 40 can be fixed, more precisely clamped, in between the masking component 70 and the frame structure 30.

The insert 48 is then placed and fixed upon the mesh substrate 40 using fixation means 50 as described hereabove. As illustrated in FIG. 13, the fixation means 50 comprise screws 61 with U-shaped flanges 72, or U-shaped washers, arranged at the screw head 62. More precisely, as illustrated in FIG. 15B, in the final configuration of the forming pocket 10, *i.e.* in an assembled state, the central portion 72a of the U, meaning the substantially flat central portion of the flange 72 that is extending horizontally and is arranged between the two branches 72b extending vertically, abuts against, or is pressed against, the screw head 62 and against two transversal plates 21, or in other words, the U-shaped flange 72 is fixed and held under tension between two consecutive, or adjacent, transversal plates 21 and the screw head 62. It can also be said that the two transversal plates 21 and the screw 61 secure the U-shape flange 72 at least axially. The second set of grid members 20 can comprise transversal plate(s) 21 comprising at least one cut-out 59 arranged at a central region (cf. FIG. 11) to ensure that the screw head does not protrudes from the lower surface of the forming pocket 11, meaning the lower surface of the grid structure 12, as illustrated in FIG. 15 thereby ensuring a good vacuum within the drum former 1.

The present disclosure is not limited to this embodiment, for instance, the U-shape flange 72 can comprise four perpendicular branches 72b so that the U-Shape flange 72 can abuts against two consecutive transversal plates 21 and two consecutive longitudinal plates 19 for better securement. According to another embodiment, the insert 48 can also comprise a threaded screw extending downwards, the fixation means 50 comprising a U-shape flange 72 as described hereabove and a threaded nut to receive said screw and affix the insert to the grid structure 12.

FIG. 14 illustrates a forming pocket 10 comprising a frame structure 30 with a grid structure 12 (not visible), a mesh substrate 40, masking component 70, a channel insert 48 and fixation means 50 (not visible) in a final configuration ready for use.

As previously discussed, the present disclosure is not limited to these embodiments. For example, according to an embodiment, the channel insert 48 can comprise integrated extended screws 61 forming a continuity of matter with said insert 48 facing, or extending, downward so that it can be fixed to the grid structure 12 by arranging fixation flanges 52 at the lower portion of some transversal plates 21 and/or longitudinal plates 19 and fixing said screws 61 to a corresponding threaded nut 56 arranged under, or below, the fixation flange 52. According to another embodiment, the fixation means 50 are fixed, or associated, to the first set of grid members 18, *i*.*e*. to the longitudinal plates 19, or to both transversal and longitudinal plates 19,21. According to another embodiment, the fixation means 50 comprises a threaded nut 56 comprising 4 extending arms, branches or hooks, each arm abutting against a transversal or longitudinal plate 19,21 so that the threaded nut 56 is arranged within the square defined by two adjacent longitudinal plates 19 and two successive transversal plates 21, each arm or hook abutting against a transversal or longitudinal plate 19,21. According to another embodiment, the threaded nut 56 can be dimensioned to fit within an air-through opening 14 defined by the grid structure 12 and be maintained by friction alone by the four plates, *i*.*e*. two transversal plates 21 and two longitudinal plates 19. In other words, the threaded nut 56 can be arranged within the grid structure 12 in a tight-fit connection. According to another embodiment, the fixation means 50 are not limited to bolts, nuts and screws, for example pins, such as linchpin, or split pin, snap-fit or hook and eye closure can also be used as fixation means 50. For example, the fixation means 50 can be a bayonet connector where the insert 48 comprises only one rod extending downward with a peg, or pin, at the end farthest from the insert 48 which can interact with a nut or connector comprising a L-shape notch that is arranged within the grid structure 12. The twisting of the insert 48 blocks the peg within the L-shaped notch and secure the insert to the grid structure 12.

The embodiments described above can be taken alone or in combination. For example, according to the present disclosure, a forming pocket 10 as described and illustrated in FIG. 6 can comprise fixation means 50 with U-shaped flanges 72. The forming pocket 10 as described and illustrated in FIG. 14 can comprise fixation means 50 with screw sleeves 54 and fixation flanges 52 arranged on transversal and/or longitudinal plates 19,21.

The forming pocket 10 according to the present disclosure enables a fast and easy change of insert 48 whether be it in shape or size with different fixation means 50. As a result, the method for making the grid supporting structure 10 and fixing the insert 48 to the grid structure 12 involves only a few constructional steps, does not require dedicated equipment and further enables passing from absorbent core having different shapes, size or channel designs by only positioning the elements 19 of the first set and the elements 21 of the second set, without the need to have different production machinery or additional processing steps.

The present disclosure also pertains to a forming apparatus for making absorbent cores for hygienic products using such forming pocket. The present disclosure also relates to an apparatus for forming moulded absorbent material deposit structures to be used as absorbent core for absorbent articles, said apparatus comprising a forming drum 1 comprising at least one forming pocket 10 as described herein, said forming pocket 10 extending in a longitudinal, transversal and vertical direction L,T,V, the forming pocket 10 being curved along the longitudinal direction L and is adapted to be fixed onto the circumference of said drum 1 and a feeding device for feeding or applying absorbent material within the forming pocket 10.

The present disclosure also pertains to a method of making forming pocket 10 as described herein, wherein the method comprises the following steps :
1. providing a first and second set of frame elements 32,34 and assembling them to define a supporting frame 30; and either
2a. providing a plurality of longitudinal plates 19 of the first set of grid member 18 and wedging them within the supporting frame 30 and providing a plurality of transversal plates 21 of the second set of grid member 20 and wedging them within the supporting frame 30 and longitudinal plates 19 of the first set of grid member 18;
2b. providing a plurality of transversal plates 21 of the second set of grid member 20 and wedging them within the supporting frame 30 and providing a plurality of longitudinal plates 19 of the first set of grid member 18 and wedging them within the supporting frame 30 and transversal plates 21 of the second set of grid member 20; or
2c. providing a plurality of longitudinal or transversal plates 19,21 of the first and second set of grid member 18,20 and wedging them together to form a grid structure 12 and wedging said grid structure 12 within the supporting frame 30; and
3. providing a mesh substrate 40 and fixing it to the grid structure 12 and/or supporting frame 30, and
4. providing an insert 48 and fixing it to said grid structure 12 via movable fixation means 50.

According to an embodiment, the method comprises a further step of providing a masking component 70 and fixing it to grid structure 12 and/or supporting frame 30, this step being prior to or after step 4 mentioned hereabove.

The present disclosure also pertains to a process for providing at least two absorbent cores with different channel designs, for an absorbent article, the process comprising the steps of:
i. providing a forming pocket 10 as described herein, said forming pocket 10 defining a mould cavity ; preferably said mould cavity being defined by the mesh substrate 40 and eventually the masking component 70n;
ii. arranging a first insert 48 into the mould cavity and engaging said first insert 48 with the grid structure 12 via the movable fixation means 50;
iii. optionally placing a non-woven within the mould cavity; e.g. said non-woven corresponding to a core-wrap commonly known in the art;
iv. inserting one or more absorbent materials into the mould cavity comprising the first insert 48 to form a first moulded absorbent material deposit structure;
v. optionally placing a second non-woven within the mould cavity on top of the absorbent materials, also a core-wrap;
vi. removing the first moulded absorbent material deposit structure from the mould cavity to form a first absorbent core comprising one or more first zones comprising a first amount of absorbent material and one or more second zones comprising a second amount of absorbent material, wherein said first and second amounts of absorbent material, each measured as a weight per unit area, are different;
vii. the first or second amount of absorbent material can be equal to 0 gsm, meaning that there is no absorbent material in the channel area,
viii. removing the first insert 48 from said mould cavity;
ix. optionally removing the mesh substrate 40 and changing the position of some longitudinal and/or transversal plates 19;21 comprising at least one fixation flange 52 and/or cut-out 59; evidently "changing the position" means changing the longitudinal position of transversal plates 21 or changing the transversal position of longitudinal plates 19;
x. arranging a second insert 48 into the mould cavity and engaging said insert 48 with the grid structure 12 via movable fixation means 50;
xi. optionally placing a non-woven within the mould cavity;
xii. inserting one or more absorbent materials into the mould cavity comprising the second insert 48 to form a second moulded absorbent material deposit structure;
xiii. optionally placing a second non-woven within the mould cavity on top of the absorbent materials;
xiv. removing the second moulded absorbent material deposit structure from the mould cavity to form a second absorbent core comprising one or more first zones comprising a first amount of absorbent material and one or more second zones comprising a second amount of absorbent material, wherein said first and second amounts of absorbent material, each measured as a weight per unit area, are different;
wherein the first and second inserts 48 are different in shape and/or dimensions for example as illustrated in FIG. 6 and FIG. 13.

## Claims

1. Forming pocket (10), suitable for forming moulded absorbent material deposit structures to be used as absorbent core for absorbent articles, the forming pocket (10), extending in a longitudinal, transversal and vertical direction (L,T,V), comprises:
- a grid structure (12) comprising a first set of grid members (18) and a second set of grid members (20), each set of grid members (18,20) comprising a plurality of plates (19,21), each plate (19,21) comprising a plurality of slits (22,26),
wherein the plates (19) of the first set of grid members (18) are configured to be wedged into the plates (21) of the second set of grid members (20) via said slits (22,26) thereby forming said grid structure (12) comprising an upper surface, a lower surface and four lateral surfaces ;
- a supporting frame (30) encasing said grid structure (12), said supporting frame (30) comprising a first set of frame elements (32) forming two opposite longitudinal walls (32) of the supporting frame (30) and a second set of frame elements (34) forming two opposite transversal walls of the supporting frame;
wherein each longitudinal walls (32) comprises a plurality of slots (35) configured to receive the extremities of the plates (19,21) of a first or second set of grid members (18,20) and/or each transversal walls (34) comprises a plurality of slots (36) configured to receive the extremities of the plates (19,21) of a second or first set of grid members (18,20);
- a mesh substrate (40) arranged on said upper surface of the grid structure (12),
and
- an insert (48) arranged on the upper surface of the mesh substrate (40), wherein the insert (48) is coupled to the grid structure (12) via movable fixation means (50).

2. Forming pocket (10) according to claim 1, wherein the movable fixation means (50) are arranged on at least one plate (19,21) of the first and/or second set of grid members (18,20).

3. Forming pocket (10) according to any of the preceding claims, wherein the movable fixation means (50) comprise a threaded fastening in which the insert (48) comprises a threaded hole and the grid structure (12) comprises a threaded screw (61) or in which the insert (48) comprises a threaded screw (61) and the grid structure (12) comprises a threaded nut (56) or in which the insert (48) and grid structure (12) both comprise a threaded hole and a stud bolt threaded at each extremities said bolt engaging both insert (48) and grid structure (12) or in which the insert (48) and grid structure (12) both comprise a threaded screw with a stud bolt comprising threaded cavities at each extremities said bolt engaging both insert (48) and grid structure (12).

4. Forming pocket (10) according to claim 3, wherein at least one plate (19,21) of the first and/or second set of grid members (18,20) comprises at least one fixation flange (52) comprising a through hole, said threaded screw (61) or bolt passing through said through hole.

5. Forming pocket (10) according to claim 4, wherein at least one plate (19,21) of the first and/or second set of grid members (18,20) comprises at least one opening (58) arranged in facing relationship to a fixation flange (52) arranged on an adjacent plate (19,21), said fixation flange (52) passing through said opening (58).

6. Forming pocket (10) according to any of the claims 1 to 3, wherein the movable fixation means (50) comprise screws (61) with U-shaped flanges (72), said plate (19,21) of the first and/or second set of grid members (18,20) comprising a cut-out (59) where the height of said plate (19,21) at said cut-out is less than the maximum height of said plate (19,21), said height corresponding to the distance along the vertical direction from one edge to the opposite edge for said plate (19,21).

7. Forming pocket (10) according to any of the preceding claims, wherein each frame elements of the first set of frame elements (32) comprises a four walls (64,66,68) defining a tube with two longitudinal walls (32) and the second set of frame element (34) defines two transversal walls (34).

8. Forming pocket (10) according to any of the preceding claims, wherein the forming pocket (10) comprises a masking component (70) arranged on the upper surface of the mesh substrate (40), said masking component (70) being attached to the supporting frame (30) and/or grid structure (12) via additional fixation means (46).

9. Forming pocket (10) according to any of the proceedings claims, wherein the mesh substrate (40) comprises a portion (40b) extending partially along at least one transversal wall (34) of the supporting frame (30), said supporting frame (30) comprising a recessed area (42) to receive said portion (40b) and/or a closing mean (45) to secure said portion (40b) to said transversal wall (34).

10. Forming pocket (10) according to any of the preceding claims, wherein each plate (19) of the first set of grid member (18) comprises a rectangular end-notch (24) arranged at each extremity of said longitudinal plate (19), and each plate (21) of the second set of grid member (20) comprises a rectangular end-notch (28) arranged at each extremity of said transversal plate (21), both longitudinal walls (32) and both transversal walls (34) comprising slots (35,36) to accommodate said extremities of each plate (19,21) respectively, said slots (35,36) being arranged at different longitudinal or transversal positions and each slots (35,36) partially extending along the vertical direction from one edge of said longitudinal wall (32) or said transversal wall (32,34) toward the opposite edge of said longitudinal or transversal wall (32,34).

11. Apparatus for forming moulded absorbent material deposit structures to be used as absorbent core for absorbent articles, said apparatus comprising:
i. a forming drum (1) comprising at least one forming pocket (10) according to any of the claims 1 to 10, said forming pocket (10) extending in a longitudinal, transversal and vertical direction (L,T,V), the forming pocket (10) being curved along the longitudinal direction (L) and being adapted to be fixed onto the circumference of said drum (1); and
ii. a feeding device for feeding absorbent material within the forming pocket (10).

12. Method of making a forming pocket (10) according to any of the claims 1 to 10, wherein the method comprises the following steps :
1. providing a first and second set of frame elements (32,34) and assembling them to define a supporting frame (30); and either
2a. providing a plurality of plates (19) of the first set of grid member (18) and wedging them within the supporting frame (30) and providing a plurality of plates (21) of the second set of grid member (20) and wedging them within the supporting frame (30) and plates (19) of the first set of grid member (18);
2b. providing a plurality of plates (21) of the second set of grid member (20) and wedging them within the supporting frame (30) and providing a plurality of plates (19) of the first set of grid member (18) and wedging them within the supporting frame (30) and plates (21) of the second set of grid member (20); or
2c. providing a plurality of plates (19,21) of the first and second set of grid member (18,20) and wedging them together to form a grid structure (12) and wedging said grid structure (12) within the supporting frame (30); and
3. providing a mesh substrate (40) and fixing it to the grid structure (12) and/or supporting frame (30), and
4. providing an insert (48) and fixing it to said grid structure (12) via movable fixation means (50).

13. Method according to claim 12, wherein the method comprises a further step of providing a masking component (70) and fixing said masking component (70) to the grid structure (12) and/or supporting frame (30), this step being prior to or after step 4.

14. Process for providing at least two absorbent cores with different channel designs, for an absorbent article, the process comprising the steps of:
i. providing a forming pocket (10) according to any of the claims 1 to 10, said forming pocket (10) defining a mould cavity ;
ii. arranging a first insert (48) into the mould cavity and engaging said first insert (48) with the grid structure (12) via the movable fixation means (50);
iii. optionally placing a non-woven within the mould cavity;
iv. inserting one or more absorbent materials into the mould cavity comprising the first insert (48) to form a first moulded absorbent material deposit structure;
v. optionally placing a second non-woven within the mould cavity on top of the absorbent materials;
vi. removing the first moulded absorbent material deposit structure from the mould cavity to form a first absorbent core comprising one or more first zones comprising a first amount of absorbent material and one or more second zones comprising a second amount of absorbent material, wherein said first and second amounts of absorbent material, each measured as a weight per unit area, are different;
vii. removing the first insert (48) from said mould cavity;
viii. optionally removing the mesh substrate (40) and changing the position of some plates (19;21) comprising at least one fixation flange (52) and/or cut-out (59);
ix. arranging a second insert (48) into the mould cavity and engaging said insert (48) with the grid structure (12) via movable fixation means (50);
x. optionally placing a non-woven within the mould cavity;
xi. inserting one or more absorbent materials into the mould cavity comprising the second insert (48) to form a second moulded absorbent material deposit structure;
xii. optionally placing a second non-woven within the mould cavity on top of the absorbent materials;
xiii. removing the second moulded absorbent material deposit structure from the mould cavity to form a second absorbent core comprising one or more first zones comprising a first amount of absorbent material and one or more second zones comprising a second amount of absorbent material, wherein said first and second amounts of absorbent material, each measured as a weight per unit area, are different;
wherein the first and second inserts (48) are different in shape and/or dimensions.
